# EUROPEAN PATENT APPLICATION

(11) **EP 1 424 650 A2**
(43) Date of publication of application: **02.06.2004**
(21) Application number: 03257456.8
(22) Date of filing: 26.11.2003
(51) Int. Cl.: G06F 19/00

(54) **Initializing model-based interpretations of digital radiographs**

(30) Priority: 27.11.2002 US 306341
(71) Applicant: CANON KABUSHIKI KAISHA, Tokyo (JP)
(72) Inventor: Tong, Xin,c/o Canon Development Americas, Inc., San José, California 95134 (US); Berestov, Alexander, c/o Canon Dev. Americas, Inc., San José, California 95134 (US)
(74) Representative: Beresford, Keith Denis Lewis

(57) **Abstract**

Automated computer aided diagnosis (CAD) processing of digital radiographs through model-based interpretation, with the initialization providing a set of initial parameters used by the model. The initial parameters can be selected based on expected pathology in the digital radiograph, and are optimized by the model to match features shown in the radiograph. The model can be an iterative model or a non-iterative model. Analysis is performed on the interpretation result, so as to diagnose pathology shown in the radiograph.

## Description

The present invention relates to computer aided diagnosis (CAD) of digital radiographs, particularly those that use deformable models in model-based interpretations of digital radiographs based on machine vision, and more particularly to initialization of such model-based interpretations.

One promising advance in the field of computer aided diagnosis (CAD) is the application of machine vision to digital radiographs. Of the various types of machine vision techniques now available, CAD often employs model-based interpretation based on deformable models of objects found in the radiographs. "Deformable models" are models that maintain the essential characteristics of the objects that they represent, such as bone and tissue structure, but deform to fit a range of examples common to multiple different radiographs of different patients.

One problem encountered with the use of model-based interpretation methods is their initialization. Specifically, many model-based interpretation methods involve iterative searches in regions local to a current estimate for the model. At each iteration, the current estimate is deformed slightly so as to provide a next iterated estimate. In general, the model converges through multiple iterations until it reaches a best estimate of the structures within the radiograph (with "best" meaning that further iterations are unlikely to lead to significantly different results).

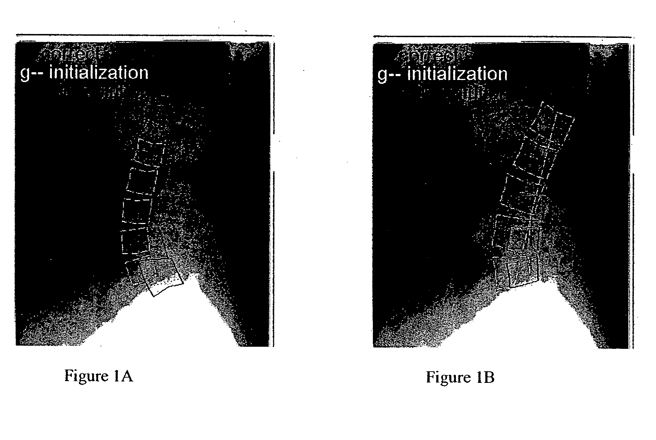

However, model-based interpretations, particularly those that involve iterative searches, are prone to significant convergence errors if they have poor initialization, for example, initialized from a bad starting location. Figures 1A and 1B illustrate this situation. Figure 1A illustrates a digital radiograph from a lateral lumbar spine examination, and further illustrates a model-based interpretation in the form of an active shape model (ASM) attributable to Cootes and Taylor. See, for example, Cootes and Taylor, "Statistical model of appearance for medical image analysis and computer vision", Proceedings SPIE Medical Imaging, pp. 236-248 (Feb. 2001). ASM employs an iterative search to deform a model from an initial position to a converged position. As seen in Figure 1A, a poor initialization position leads to convergence at an incorrect estimate of the position of the lumbar vertebrae. On the other hand, as seen in Figure 1B, a good initialization position leads to correct convergence at accurate locations of the lumbar vertebrae.

Good initialization parameters can be obtained by manual input by skilled radiologists or other medical personnel. However, manual initialization is counterproductive to the goals of a fully automated CAD procedure.

It is an object of the invention to address the foregoing situation through an automated analysis to obtain initial values for model parameters for a model-based interpretation of a digital radiograph. The invention is based on the recognition of the inventors herein that all radiographs obtained in accordance with any one specific radiographic protocol (for example, a lateral lumbar spine examination) will include distinctive regions common from patient-to-patient and from examination-to-examination.

Thus, according to one aspect, the invention obtains initial values for model parameters for a model-based interpretation of a digital radiograph obtained from a patient in accordance with a radiographic protocol. A region of interest in the radiograph is identified, based on landmarks common to multiple different radiographs obtained with the same radiographic protocol. The region of interest is thereafter analyzed so as to calculate candidates for the initial model parameters. If needed, the candidates can be thereafter refined so as to disambiguate candidates with respect to repetitive structures found in the region of interest.

In preferred embodiments, the landmarks comprise distinctive regions of high contrast within each different radiographic image produced by the same radiographic protocol. For example, in the case of a lateral lumbar spine protocol, radiographic images are characterized by a bright pelvic area, a dark area corresponding to a non-patient region beyond the patient's back, and a dark lung area. The dark lung area is separated from the dark non-patient region by a bright spine comprising the region of interest. These landmarks together are identified using image enhancement techniques such as equalization, window leveling, and thresholding so as to define the region of interest.

In further preferred embodiments, candidates for model parameters are calculated based on visually significant features in the region of interest together with spatial orientation of such features within the region of interest. Since the model might often contain repetitive structures which are difficult to disambiguate within the region of interest, the candidates are calculated merely to find some of the repetitive structures without distinguishing one from the other. For example, in a lateral lumbar spine examination, the region of interest might consist of five vertebrae above the iliac bone. Initial parameters for a deformable model might therefore consist of parameters that define five nearly-identical rectangular regions, and it is therefore difficult to distinguish one of the vertebrae (and corresponding rectangular region) from another. Accordingly, the candidates often do not distinguish between the repetitive structures and often might contain a shift of one up or down from the actual position in the radiograph.

Disambiguation is preferably performed relative to the landmarks used to determine the region of interest, as well as relative to boundaries of the region of interest itself. For example, once significant vertebrae are identified in the region of interest for a lateral lumbar spine examination, and candidates calculated based on the significant vertebrae, the candidates can be disambiguated by distance measurements relative to the dark region of the lung and the bright region of the iliac bone.

The initial values of the parameters may correspond to expected pathology in the radiograph. For example, in a situation where normal pathology is expected, a "normal" initial model can be selected. Likewise, in a situation where an abnormal pathology is expected, an "abnormal" initial model can be selected. Alternatively, processing according to two or more of multiple different sets of initial parameters can be conducted, with automated selection of one of them being based on convergence of the deformable model.

The model-based interpretation may be an interpretation based on deformable models, and can be an iterative model such as ASM described by Cootes and Taylor, and an active appearance model (AAM), also described by Cootes and Taylor. Alternatively, the model-based interpretation, might be a non-iterative model such as a model implemented through neural networks or wavelet analysis of digital radiographic content.

Further preferred embodiments of the invention involve automated CAD processing of a digital radiograph through calculation of initial values for model parameters for a model-based interpretation of the digital radiograph, followed by revision of the initial values according to the model-based interpretation so as to obtain a best estimate of parameters that accurately model features found within the radiograph. Measurements are then obtained from the interpretation results so as to provide computer assisted diagnosis of pathology found in the radiograph. For example, in the case of a lateral lumbar spinal examination, CAD processing can be performed so as to diagnose kyphosis and lordosis, together with a quantification of the relative severity of these conditions.

This brief summary has been provided so that the nature of the invention may be understood quickly. A more complete understanding of the invention can be obtained by reference to the following detailed description of the preferred embodiment thereof, which is described by way of example only, in connection with the attached drawings in which:
Figures 1A and 1B are representative digital radiographs showing the effects of poor initialization versus good initialization;
Figure 2 is a block diagram showing a teleradiological computer aided diagnostic (CAD) system;
Figure 3 is a flow diagram showing CAD analysis according to an embodiment of the invention;
Figures 4A and 4B are views for explaining designation of training points in digital radiographs;
Figure 5 is a view explaining automated search of image data in a digital radiograph so as to obtain accurate modeling of shapes therein;
Figure 6 is a detailed flow diagram showing a method for obtaining initial values for model parameters according to an embodiment of the invention;
Figures 7A through 7F are representative digital radiographs of different patients under the same radiological protocol;
Figures 8A through 8F are views for explaining image processing by which a region of interest is identified;
Figures 9A through 9F are views for explaining image processing by which candidates are calculated for initial model parameters;
Figures 10A through 10F are views of the same digital radiographs shown in Figures 7A through 7F, but with identified vertebrae mapped onto the image, for explaining disambiguation according to an embodiment of the invention; and
Figure 11 is a flow diagram for explaining a second embodiment of the invention.

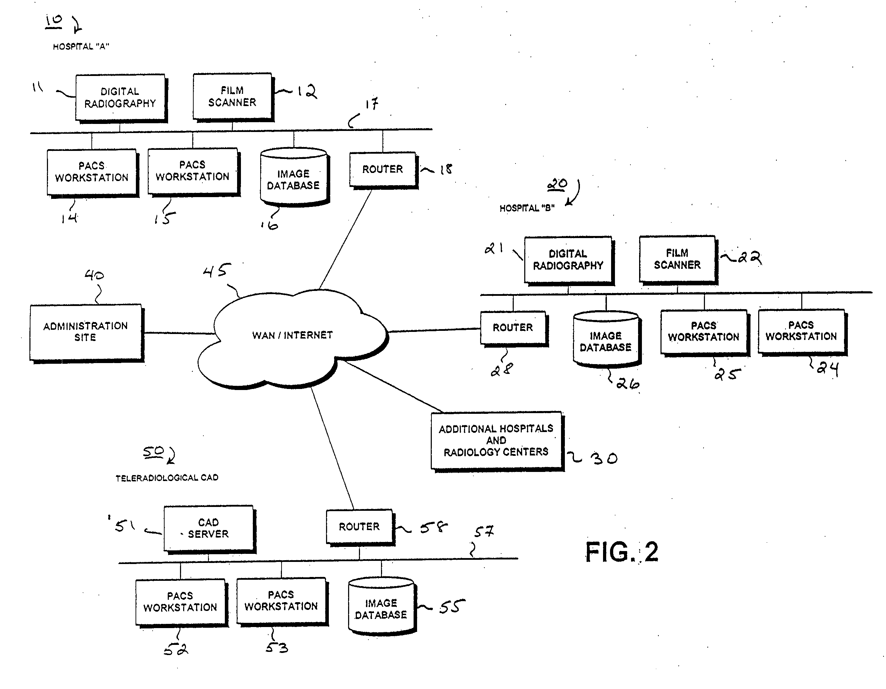

Figure 2 is a generalized block diagram view of a teleradiological CAD (computer aided diagnosis) system. As shown in Figure 2, a teleradiological CAD system includes multiple hospitals and radiology centers 10, 20 and 30, an administrative site 40, and a teleradiological CAD site 50, all interconnected through a wide area network 45 or over the network. A typical hospital includes digital radiography equipment 11 for obtaining original digital radiographs from a patient, as well as a film scanner 12 for converting film x-rays into digital radiographic form. The hospital further includes PACS (picture archiving and communication system) workstations 14 and 15, all of which intercommunicate with image database 16 over a network connection 17 which may be a local area network, a wide area network, or an intranet. A router 18 provides communications with other components of the teleradiological CAD system.

Hospital 20 and the additional hospitals and radiology centers 30 include similar architecture, although it is to be understood that these architectures are illustrative only of the general nature of radiology centers.

Administrative site 40 administers the teleradiological CAD aspects of the system such as by accepting and routing requests to appropriate CAD sites as well as routing diagnostic information back to the requesting site, as appropriate.

Teleradiological CAD site 50 includes a CAD server 51 which communicates with PACS workstations 52 and 53 as well as image database 55 over a network connection 57. Router 58 interconnects the teleradiological CAD site 50 to other components of the system.

In a representative operational aspect, hospital 10 will obtain a digital radiograph using digital radiography equipment 11 or film scanner 12, which is stored on image database 16. A radiologist or other medical personnel using one of the PACS workstations 14 or 15 issues a request for CAD services, which is handled by administrative site 40. Administrative site 40 routes the request to teleradiological CAD site 50 where CAD server 51 services the request. Technicians at CAD site 50 are preferably involved with the CAD analysis using one of PACS workstations 52 and 53. The image data itself might be obtained form image database 16, or might be transferred to image database 55. Preferably, the image is stored in DICOM format. The results of CAD analysis is routed back to hospital 10 through administrative site 40, where it reaches the original requestor at one of PACS workstations 14 and 15.

In this embodiment, the CAD system of the present invention resides on CAD server 51. Of course, other embodiments are possible, such as embodiments where CAD is performed locally in PACS workstations 14 or 15, or on the digital radiography equipment 11 itself. However, the centralized approach of the present embodiment has advantages over distributed approaches, such as the advantage of providing uniform diagnosis as well as the advantage of simplified update in the event of an upgrade in automated diagnostic capabilities.

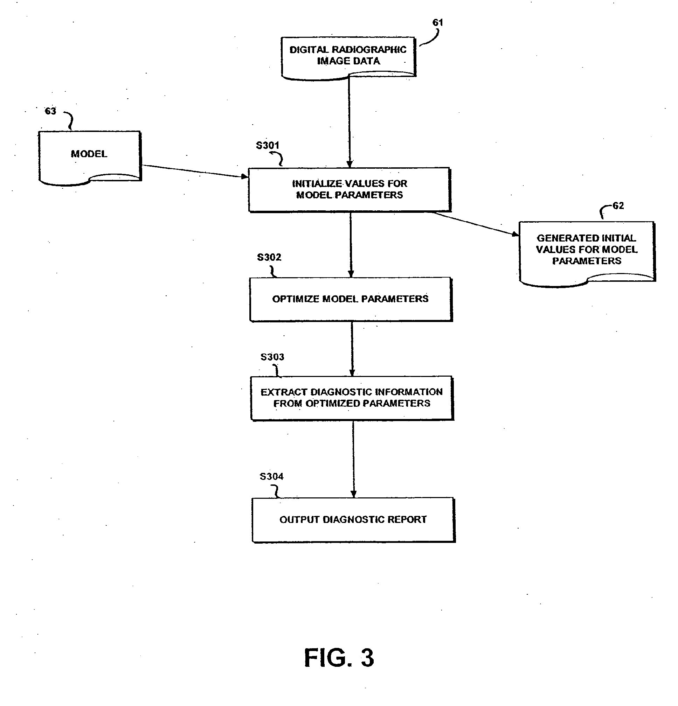

Figure 3 is a generalized flow diagram showing CAD processing in CAD server 51. CAD processing according to the invention is a model-based interpretation of digital radiographs in which a plurality of model parameters define a mathematical model of features in the digital radiograph. Presently, two models are preferred, both attributable to Cootes and Taylor: the aforementioned active shape model (ASM) in which shapes are modeled by matching grey-level intensity characteristics around model points so as to achieve a best match to corresponding points in training data; and active appearance model (AAM) in which shape and texture are used to constrain object appearance during the search. Both ASM and AAM employ iterative searches so as to achieve a best match, and other iterative model-based interpretations can be employed. In addition, non-iterative model-based interpretations can also be employed, such as neural networks and the like.

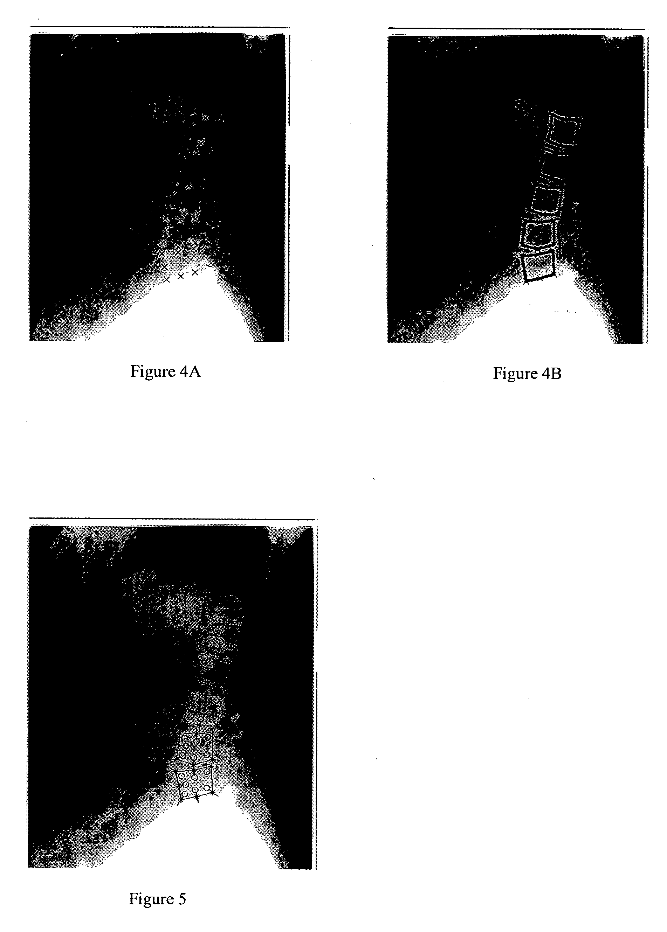

As shown in Figure 3, the initial values of the model parameters are calculated in step S301 using image data 61 representing the digital radiograph being subjected to CAD processing, as well as the model 63. The model is based on training data which is empirically derived data obtained beforehand, usually through analysis of a large number of digital radiographs from the same radiographic protocol as that represented by image data 61. The empirical analysis is typically undertaken by skilled physicians by the means of hand annotations of training images. The physicians draw landmark points in each of the training images, then additional points along boundaries are generated by interpolation between the landmark points. Figures 4A and 4B illustrate how a training shape is generated by this method, using the example of ASM. The image in Figure 4A shows landmark points that a skilled physician draws by hand, whereas the image shown in Figure 4B shows the shape resulting from interpolating along boundaries.

Although annotation of hundreds of training imagesby hand using skilled physicians is a time-consuming task, it is preferred to automatic or semi-automatic methods of annotation since the accuracy and reliability of skilled physicians yields improved training data.

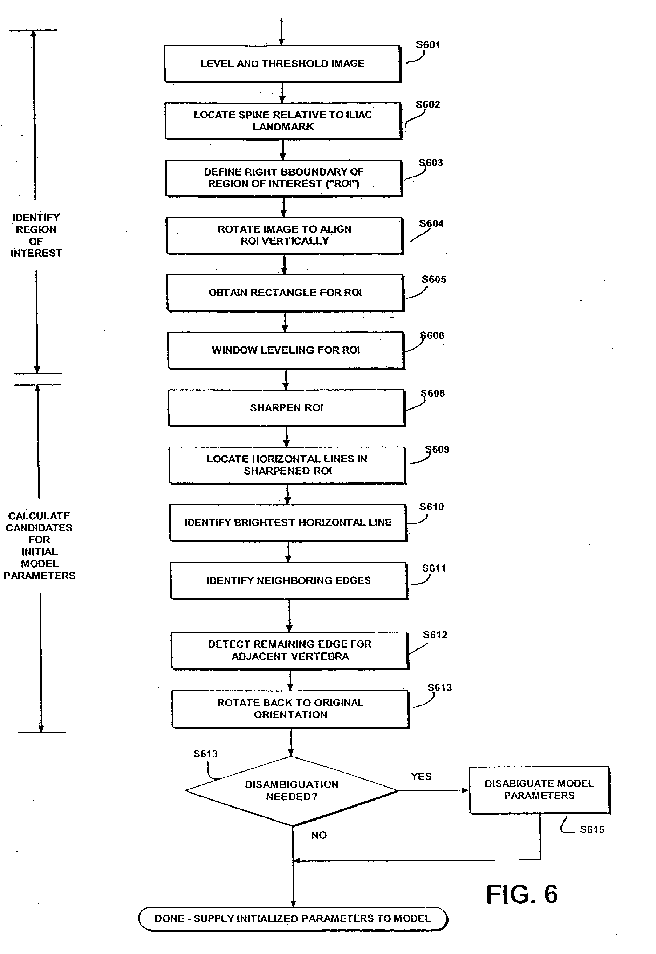

Reverting to Figure 3, Step S301 generates initial values for the model's parameters as explained more fully below in connection with Figure 6. The generated initial values are shown diagrammatically at reference numeral 62. After initialization in step S301, model parameters are optimized in step S302. The precise method of optimization depends on the particular model-based interpretation being employed. In the case of ASM, an iterative search is performed so as to inspect changes in image intensities along profiles normal to the model boundary through each model point. For a given model point, the grey-level intensity (or its derivative) is sampled along a profile of k pixels on either side of the point in the image. This is illustrated in Figure 5, which shows line segments extending outwardly from "0" marks, and along which image intensity characteristics are sampled. A multivariate Gaussian model of grey-level intensity (or derivative) samples is created for each model point using profiles from the training set. During the ASM search, each model point is moved along the profile to achieve a "best" match in grey-level intensity characteristics, with "best" meaning that no significant improvements in accuracy are obtained through further iterations. Intuitively, if the model boundary corresponds to an edge, the aforementioned search process will locate the most similar edge along the profile. After updating all model point positions, new model parameters are found to fit the model shape to the new shape. This process, of moving model points to best match the imaging characteristics and then updating the model shape parameters, repeats until convergence is achieved since no significant change in point positions is obtained with further iterations.

A similar approach is undertaken using model-based interpretation according to AAM, although AAM uses not only the shape of features within an image but also uses the description of texture across the object.

After model parameters have been optimized in step S302, step S303 operates to extract diagnostic information from the optimized parameters. For example, in the case of a radiographic protocol of a lateral lumbar spine examination, automatic measurements are made of disk space, vertebral height, etc. Extraction of diagnostic information such as by automatic measurement proceeds based on the interpretive model used as well as the radiographic protocol employed. For example, in radiographic protocols involving the forearm, foot, or hand, infant hip, infant foot, and leg x-rays, automatic measurements are made of significant image characteristics common to those protocols. As a further example, in the case of three-dimensional radiographic images, three-dimensional measurements may be made such as measurements that might differentiate between scoliosis, kyphosis and lordosis. Finally, in step S304, a diagnostic report is output.

All model-based interpretations of imagery need good initialization of model parameters so as to ensure convergence. ASM starts with an initial shape, based on which imaging characteristics are extracted and used to improve the shape. AAM starts with some initial appearance model parameters, which provide both initial shape and initial texture.

Figure 6 is a flow diagram showing initialization of model-based interpretations according to an embodiment of the invention (step S301 in Figure 3). Briefly, Figure 6 illustrates a technique for obtaining initial values for model parameters in a model-based interpretation of a digital radiograph obtained from a patient in accordance with a radiographic protocol, in which the model-based interpretation revises the initial values to obtain revised values for the parameters based on content of the digital radiograph so as to model features therein. As shown in Figure 6, a region of interest is identified in the radiograph, wherein the region of interest is identified based on landmarks common to multiple different radiographs obtained from the same radiographic protocol. The region of interest is thereafter analyzed so as to calculate candidates for the initial model parameters. If the candidates are unambiguous, then the candidates are used as the initial model parameters for the flow shown in Figure 3. On the other hand, if the candidates are ambiguous, then the candidates are disambiguated with respect to repetitive structures found in the region of interest.

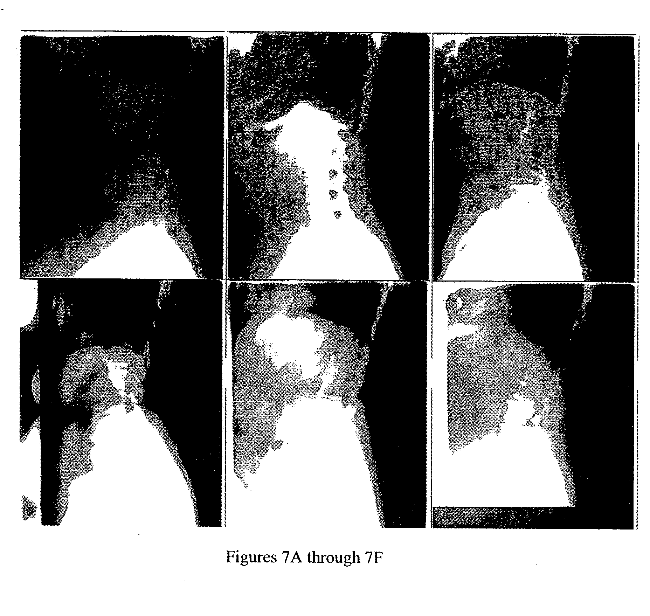

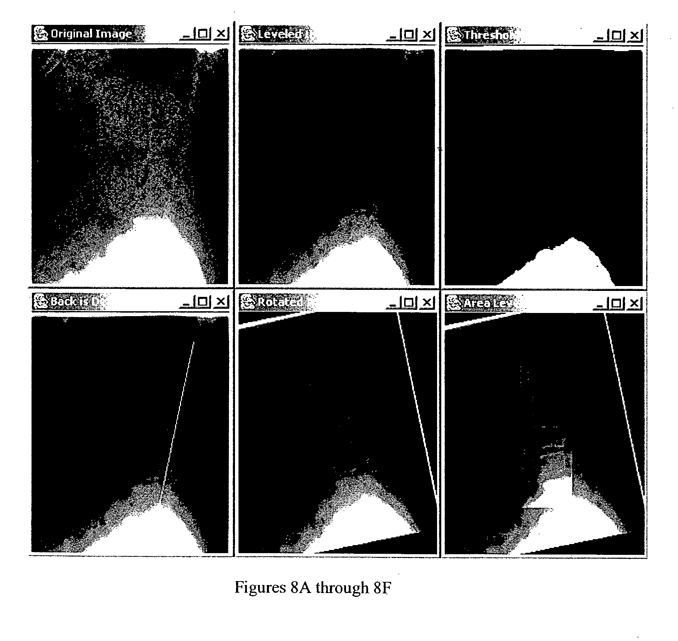

Turning more specifically to Figure 6, the inventors herein have recognized that all radiographs obtained in accordance with any one specific radiographic protocol (for example, a lateral lumbar spine examination), will include distinctive regions common from patient-to-patient and from examination-to-examination. These distinctive regions form landmarks common to multiple different radiographs, and these landmarks can be used to identify a region of interest in any one particular radiograph. For example, Figures 7A through 7F illustrate different radiographs from different patients all for the same protocol, which, in this case, is a lateral lumbar spine examination. Although the patients are different and the examinations are different, each of Figures 7A through 7F include common landmarks in the form of a bright pelvic area, a dark area corresponding to a non-patient region beyond the patient's back, and a dark lung area. These three areas surround the lumbar spine, which is the region of interest for this particular radiographic protocol. Identification of the region of interest based on these common landmarks is described in more detail in connection with steps S601 through S606. First, recognizing that original images are rarely perfect, window leveling is performed in step S601. This is illustrated in Figure 8B in connection with an original digital radiograph shown in Figure 8A (which also corresponds to the radiograph shown in Figure 7A). Thresholding is also applied in step S601 (shown in Figure 8C) with the intent being to locate the iliac bone. The spine is detected in the top part of the image, adjacent the dark lung area (step S602). The right boundary of the region of interest is then detected by connecting the right boundary of the spine with the top of the iliac bone (step S603), as illustrated in Figure 8D. The image is rotated in step S604 in order to make the boundary vertical, which provides simplified processing based on horizontal and vertical lines and structures. A rotated image is shown in Figure 8E. A rectangular region of interest is then obtained based on empirically derived information concerning the radiographic protocol. In the case of a lateral lumbar spine examination, it has been determined that suitable regions of interest are approximately 1/4 of the image width, and based on the lower level of the lung bottom, a rectangular region of interest is obtained for the lumbar vertebrae. Additional window leveling is performed within the region of interest to accentuate features therein (step S606). The leveled region of interest in depicted in Figure 8F.

After the region of interest is identified, steps S608 through S613 operate to analyze the region of interest so as to calculate candidates for the initial model parameters. In the case of a lateral lumbar spine examination, the model parameters define the shape of five vertebrae within the region of interest. Since these vertebrae are in relatively fixed position to each other, the center of one vertebra in the image provides sufficient information to obtain candidates for the model parameters. Extra information about the vertebrae can further improve initialization of model parameters.

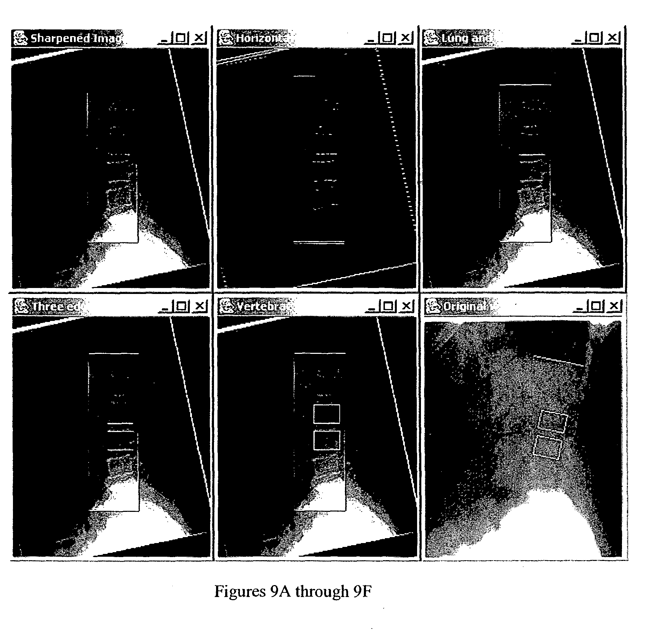

Thus, in step S608, the image within the region of interest is sharpened, and a simple 3x3 filter mask is applied to locate horizontal lines (step S609). The results of these steps are depicted in Figures 9A and 9B, respectively. A brightest horizontal line is found, and its width calculated (step S610), this brightest horizontal line being assumed to correspond to a vertebral edge. Then, two other neighboring edges are detected (step S611). The edge detected depends on the location of the brightest vertebra relative to the lung and the iliac bone. In particular, the neighboring edges are searched downward to the first edge, or upward to the first edge, or one up and one down depending on this location. In any of these cases, the original edge plus the two neighboring edges define one vertebra and the edge of a vertebra adjacent thereto. Results of edge detection is shown in Figure 9D.

A fourth edge is then detected (step S612) so as to outline two adjacent vertebrae, as shown in Figure 9E. These edges are rotated back to the original orientation (step S613), as shown in Figure 9F. The center of one of the outlined vertebrae could be chosen to initialize the model parameters.

With many images, the resulting approximate location is sufficient to define candidates for the initial model parameters. For example, when applied to a radiographic protocol involving cartilage of the knee, the shape defined by the initial model parameters is sufficiently distinct that the candidates are usable for the initial model parameter. However, in other circumstances, particularly one in which there are multiple repetitive structures that are similar to each other, disambiguation may be necessary to disambiguate the candidates with respect to these repetitive structures.

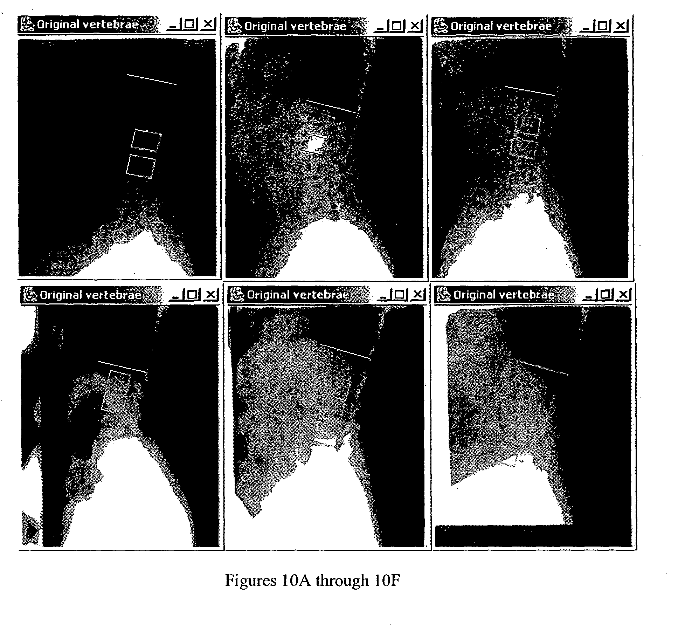

That situation presents itself in lateral lumbar spinal examinations, where the five vertebrae are repetitive rectangles that are often difficult to distinguish. Figures 10A through 10F illustrate this situation, in which steps S601 through S613 have been applied to the six digital radiographs originally illustrated in Figures 7A through 7F. As seen in Figures 10A through 10F, steps S601 through S613 have resulted in candidates that identify different vertebrae in each patient. As a consequence, disambiguation is needed for this protocol.

If disambiguation is needed, it is performed in step S615. In the case of lateral lumbar spinal examinations, disambiguation is performed by identifying each vertebra using its relative location to the lung and iliac bone.

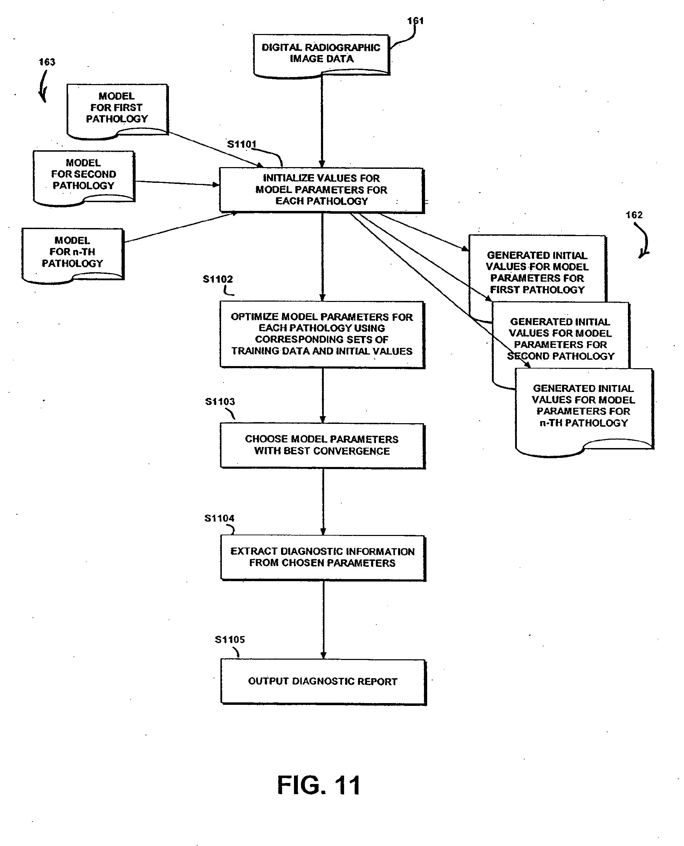

Figure 11 illustrates a second embodiment of the invention in which multiple different sets of initial parameters are generated, one each for respectively different pathologies in the radiographic protocol in questions. Likewise, multiple different models are provided, one for each of the different sets of pathologies. For example, in the case of a lateral lumbar spinal examination, different pathologies can include scoliosis, kyphosis and lordosis. The model-based interpretation uses associated models and initialized model parameters so as to obtain a corresponding multitude of converged model parameters (step S1102). Each set is analyzed to determine which has converged the best, and converged model parameters for that set are chosen (step S1103). For the chosen convergence, diagnostic information is extracted (step S1104) and a diagnostic report is output (step S1105).

The invention has been described with respect to particular illustrative embodiments. It is to be understood that the invention is not limited to the above-described embodiments and that various changes and modifications may be made by those of ordinary skill in the art without departing from the scope of the invention.

## Claims

1. A method for obtaining initial model parameters for a model-based interpretation of a digital radiograph obtained from a patient in accordance with a radiographic protocol, wherein the model-based interpretation changes the model parameters based on content of the digital radiograph so as to model features therein, said method comprising:
identifying a region of interest in the radiograph, wherein the region of interest is identified based on landmarks common to multiple different radiographs obtained with the same radiographic protocol; and
analyzing the region of interest so as to calculate one or more candidates for initial model parameters.

2. A method according to Claim 1, wherein the landmarks comprise distinctive regions of high contrast within multiple different radiographic images produced by the same radiographic protocol.

3. A method according to Claim 2, wherein the radiographic protocol is a lateral lumbar spine protocol, and wherein the landmarks include a bright pelvic area, a dark area corresponding to a non-patient region beyond the patient's back, and a dark lung area, which is separated from the dark non-patient area by a bright spine comprising the region of interest.

4. A method according to Claim 1, wherein said identifying step comprises image enhancement techniques including equalization, window leveling, and thresholding so as to define the region of interest.

5. A method according to Claim 1, wherein the candidates for initial model parameters are calculated based on visually significant features in the region of interest together with spatial orientation of such features within the region of interest.

6. A method according to Claim 5, wherein the radiographic protocol is a lateral lumbar spine examination, and the initial parameters for the deformable model define five nearly-identical rectangular regions corresponding to five vertebrae above the iliac bone, and the candidates for initial model parameters are calculated without regard to which of the five rectangular regions corresponds to one of the vertebrae.

7. A method according to Claim 1, further comprising the step of disambiguating the candidates for initial model parameters with respect to repetitive structures found in the region of interest.

8. A method according to Claim 7, wherein disambiguation is performed relative to the landmarks used to determine the region of interest.

9. A method according to Claim 8, wherein disambiguation is also performed relative to boundaries of the region of interest itself.

10. A method according to Claim 9, wherein the radiographic protocol is a lateral lumbar spine examination, and the initial model parameters are disambiguated by distance measurements relative to a dark region of the lung and a bright region of the iliac bone.

11. A method according to Claim 1, further comprising the step of selecting an initial set of model parameters from among multiple different initial sets corresponding to multiple different pathologies.

12. A method according to Claim 1, further comprising the steps of:
selecting multiple different models corresponding to multiple different pathologies;
obtaining an initial set of model parameters for each different model according to Claim 1;
changing each initial set of model parameters according to the model-based interpretation; and
selecting one set of model parameters based on convergence of all models in the model-based interpretation.

13. A method according to Claim 1, wherein the model-based interpretation is based on an iterative model.

14. A method according to Claim 1, wherein the model-based interpretation is based on a non-iterative model.

15. Automated CAD processing of a digital radiograph through identification of a set of initial model parameters for a model-based interpretation of the digital radiograph according to any of Claims 1 to 14, comprising:
changing model parameters according to the model-based interpretation so as to obtain a best estimate of features found within the radiograph; and
analysis of the interpretation results so as to provide computer assisted diagnosis of pathology found in the radiograph.
